# EUROPEAN PATENT SPECIFICATION

(11) **EP 1 171 418 B1**
(45) Date of publication and mention of the grant of the patent: **29.12.2004**
(21) Application number: 00913489.1
(22) Date of filing: 16.02.2000
(51) Int. Cl.: C07C 227/08, C07C 229/58

(54) **METHOD FOR MAKING 2-(N-PHENYLAMINO)BENZOIC ACIDS**
VERFAHREN ZUR HERSTELLUNG VON 2-(N-PHENYLALMINO)BENZOESÄURE
PROCEDE DE PREPARATION D'ACIDES 2-(N-PHENYLALMINO)BENZOIQUES

(30) Priority: 21.04.1999 US 130384 P
(43) Date of publication of application: 16.01.2002
(73) Proprietor: Warner-Lambert Company LLC, Morris Plains, New Jersey 07950 (US)
(72) Inventor: CHEN, Michael, Huai, Gu, Ann Arbor, MI 48105 (US); MAGANO, Javier, Ypsilanti, MI 48197 (US)
(74) Representative: Tesch, Rudolf, Dr.
(86) International application number: PCT/US2000/003982
(87) International publication number: WO 2000/064856

(56) References cited:
- WO-A-99/01426

## Description

### FIELD OF THE INVENTION

The present invention relates to a method for making 2-(N-phenylamino)benzoic acids by coupling a benzoic acid and an aniline.

### BACKGROUND OF THE INVENTION

The compound 2-(2-chloro-4-iodo-phenylamino)-N-cyclopropylmethoxy-3,4-difluoro-benzamine is being developed as a selective MEK-1 inhibitor for the treatment of proliferative diseases, including cancer, restenosis, psoriasis, and atherosclerosis. See, for example, US Patent Application Number 60/051,440, filed July I, 1997, or PCT Published patent Application Number WO 99/01426, published January 14, 1999, which are hereby incorporated by reference. To make 2-(2-chloro-4-iodo-phenylamino)-N-cyclopropylmethoxy-3,4-difluoro-benzamine, one of the intermediates that is needed is a 2-(N-phenylamino) benzoic acid. The present invention provides a method for making 2-(N-phenylamino)benzoic acids.

### SUMMARY OF THE INVENTION

The present invention provides a method for making 2-(N-phenylamino)benzoic acids, the method comprising the step of reacting a benzoic acid having the Formula I 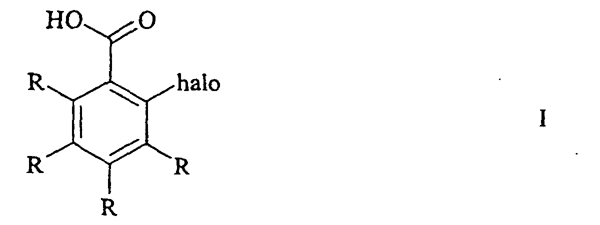 and an aniline having the Formula II 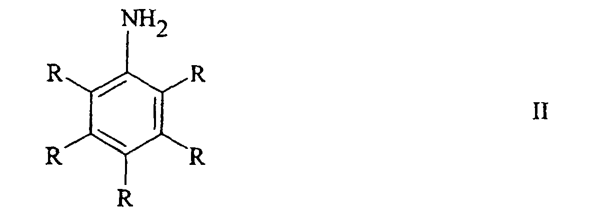 with an alkaline metal hexamethyldisilazide to form a 2-(N-phenylamino)benzoic acid having the Formula III 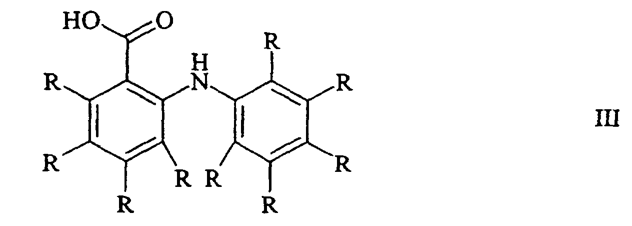 wherein
each R is independently hydrogen, halogen, C₁-C₆ alkyl, -OC₁-C₆ alkyl, CN, or
NO₂, with the proviso that R on the aniline is not NO₂.

In a preferred embodiment of the invention, the alkaline metal hexamethyldisilazide is lithium hexamethyldisilazide (LiHMDS).

In another preferred embodiment of the invention, the alkaline metal hexamethyldisilazide is 3 equivalents or more with respect to the benzoic acid.

In another preferred embodiment of the invention, the halo substituent in the 2 position of the benzoic acid is fluorine.

In another preferred embodiment of the invention, the reaction is carried out at -78°C to 25°C in a polar, aprotic solvent.

In a more preferred embodiment, the solvent is tetrahydrofuran.

In another preferred embodiment of the invention, the benzoic acid is 2,3,4,-trifluorobenzoic acid and the aniline is 2-chloro-4-iodoaniline.

In another preferred embodiment of the invention, the benzoic acid and the aniline are present in a 1:1 molar ratio.

In another preferred embodiment of the invention, one or more of the substituents R on the aniline is an electron donating group.

In another preferred embodiment of the invention, the electron donating group on the aniline is -OCH₃.

### DETAILED DESCRIPTION OF THE INVENTION

The present invention provides a method for making 2-(N-phenylamino)benzoic acids. The method comprises the coupling of a benzoic acid having the Formula I 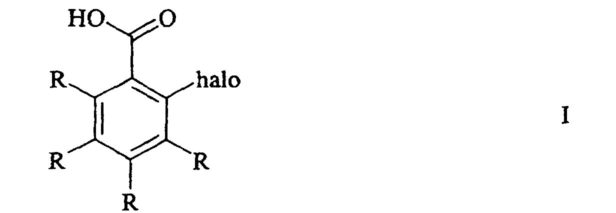 and an aniline having the Formula II 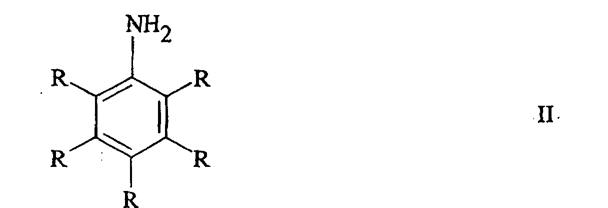 using an alkaline metal hexamethyldisilazide as a base to form a 2-(N-phenylamino)benzoic acid having the Formula III

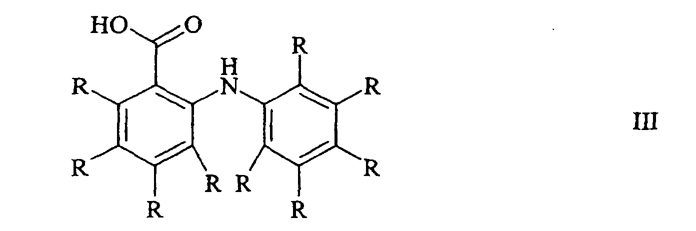

This coupling reaction preferably uses 1 equivalent each of the benzoic acid and the aniline. Thus, the molar ratio of the benzoic acid to the aniline is 1:1. In addition, 3 equivalents of the alkaline metal hexamethyldisilazide is preferred; however, it is possible to use more than 3 equivalents of the alkaline metal hexamethyldisilazide. In other words, 3 moles of alkaline metal hexamethyldisilazide to every 1 mole of benzoic acid or aniline is typically used. Lithium hexamethyldisilazide is also known as lithium bis(trimethylsilyl)amide, which can be purchased from Aldrich, Milwaukee, WI.

The selection of an alkaline metal hexamethyldisilazide as a base is important because this base provides for an unexpected and surprising increase in the yield of the resulting 2-(N-phenylamino)benzoic acid when compared with other bases that are not alkaline metal hexamethyldisilazides. A most preferred alkaline metal hexamethyldisilazide is lithium hexamethyldisilazide.

The coupling reaction of the benzoic acid and the aniline can be carried out in a single pot process or in a multiple pot process. Other methods and sequences for carrying out the coupling reaction can be easily determined by those skilled in the art. Three procedures are specifically illustrated below.

The first procedure, called Method A, is a two-pot procedure. In a first flask, LiHMDS (1 equivalent) was added to a solution of the benzoic acid (1 equivalent) in tetrahydrofuran (THF) at -78°C. In a second flask, LiHMDS (2 equivalents) was added to a solution of the aniline (1 equivalent) in THF at -78°C. The contents from the first flask were transferred into the second flask and the resulting mixture allowed to reach ambient temperature overnight. The product was then purified by flash column chromatography.

The second method, called Method B, is a one-pot procedure. Both the benzoic acid (1 equivalent) and the aniline (1 equivalent) were dissolved in THF. The solution was cooled to -78°C and the LiHMDS added, and the mixture was allowed to warm up to ambient temperature overnight. The product was then purified by flash column chromatography.

The third method, called Method C, is a two-pot procedure. Method C is similar to Method A, except that the 3 equivalents of LiHMDS are added to the aniline followed by a solution of the benzoic acid in THF.

The results of the coupling of various benzoic acids with various anilines is shown in Table 1 below.

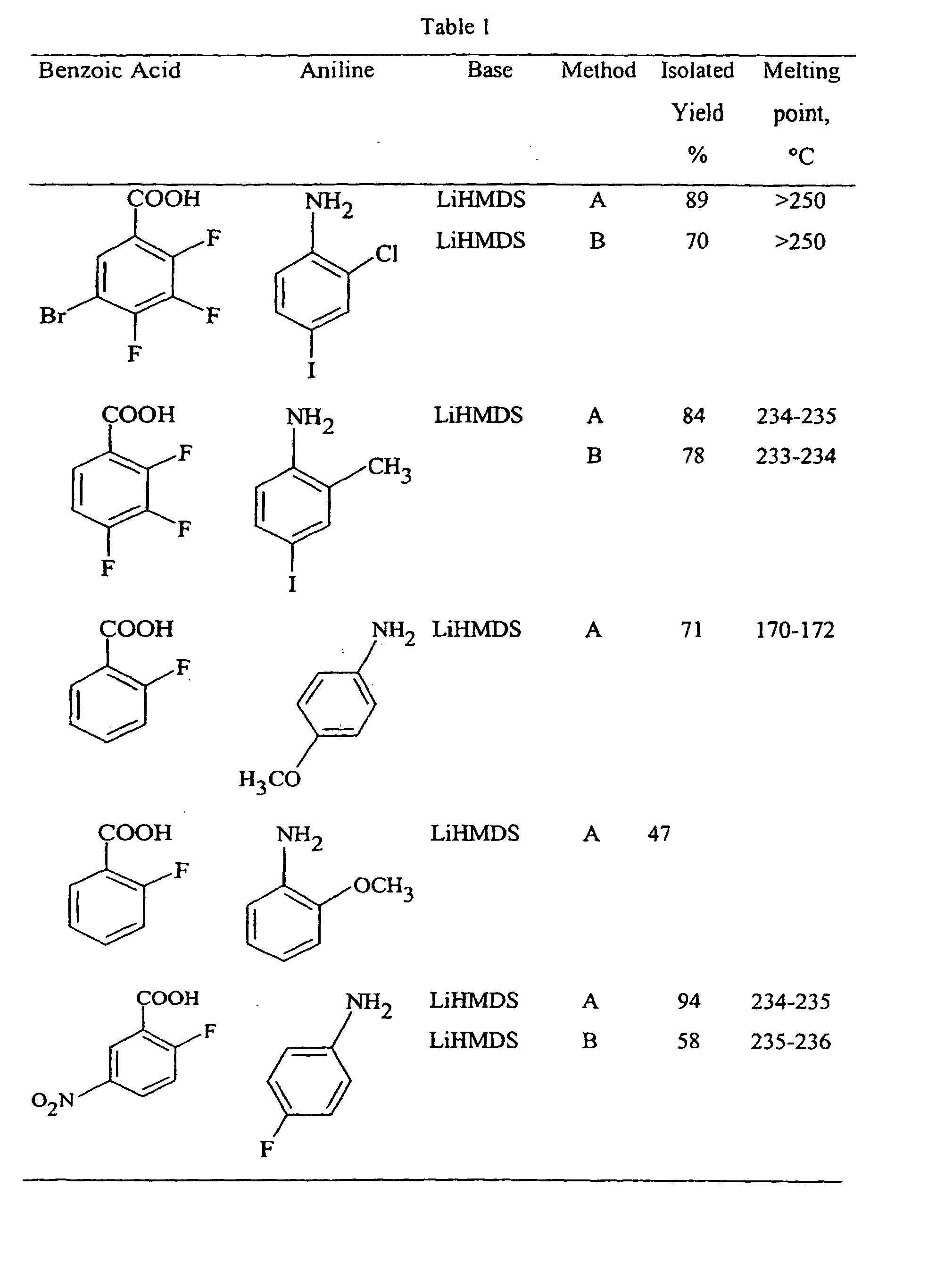

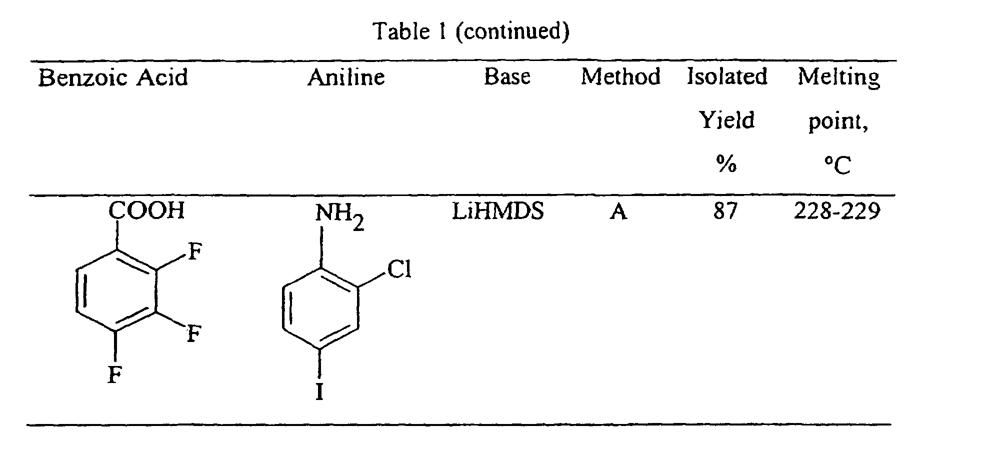

Alkali metal hexamethyldisilazide bases gives unexpectedly superior yields of the 2-(N-phenylamino)benzoic acid when compared with other bases that are not alkali metal hexamethyldisilazides. For example, in the reaction between 2,3,4-trifluorobenzoic acid and 4-iodo-2-methylaniline, the yields were 84% using LiHMDS and only 28% using lithium diisopropylamine (LDA). Moreover, no reaction was observed using NaH or triethylamine (TEA). The results of these comparisons are shown in Table 2 below.

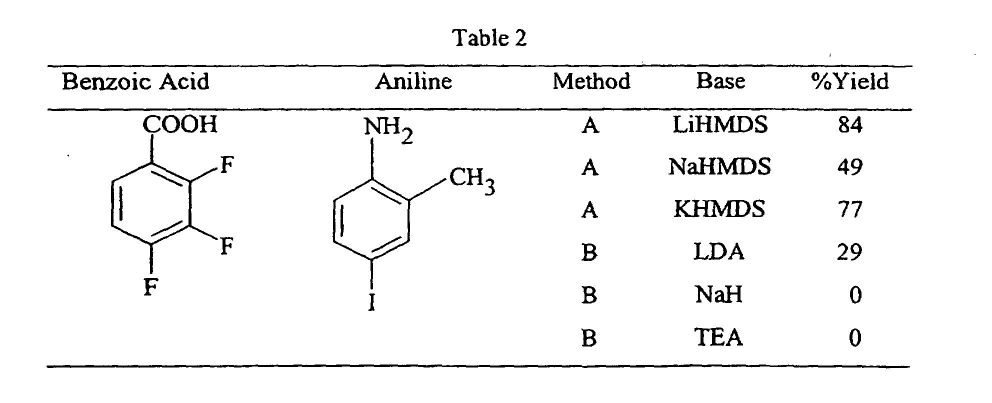

In addition, the amount of base used is important. Reducing the number of equivalents from 3 to 2 decreases the yield of the 2-(N-phenylamino) benzoic acid. The results are shown in Table 3. The use of more than 3 equivalents of base did not have a significant effect on the yield.

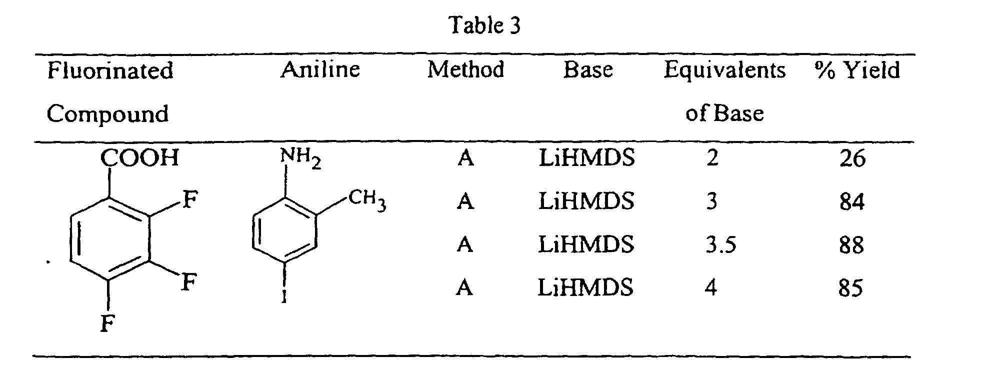

The position of the halogen atom with respect to the carboxylic acid group on the benzoic acid is also important. For example, only 2-fluorobenzoic acids reacts with anilines to give the appropriate 2-(N-phenylamino)benzoic acid. Table 4 shows the results of variation of the position of the halogen atom with respect to the carboxylic acid group on the benzoic acid.

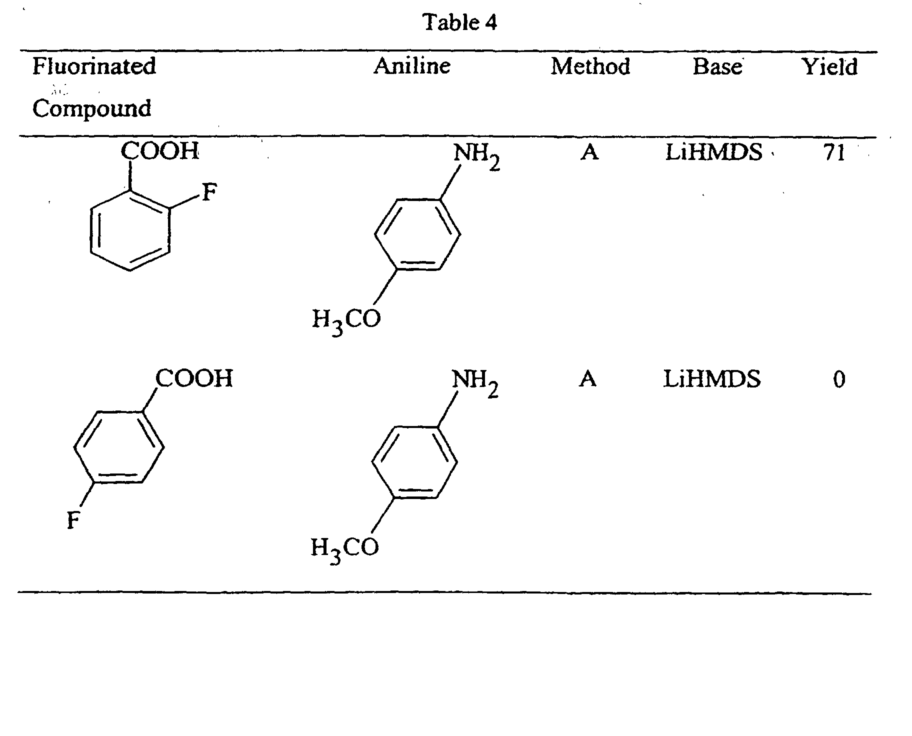

Thus, the reaction between 2-fluorobenzoic acid and *p*-anisidine afforded 71% of the desired product. In contrast, when 4-fluorobenzoic acid was employed, no reaction was observed.

The substituents on the aniline ring also affect the yield of the resulting 2-(N-phenylamino)benzoic acid. For example, the presence of electron-donating groups, such as -OC₁-C₆ alkyl, halogen, C₁-C₆ alkyl, dialkylanimes, or -SC₁-C₆ alkyl, and others well-known to those skilled in the art, increases the reactivity of the aniline and results in a higher yield of the 2-(N-phenylamino)benzoic acid. If electron-withdrawing groups such as nitro, halogen, carbonyl (both aldehyde and ketone), ester and nitrile, and others well-known to those skilled in the art, are substituents on the aniline, the reactivity decreases and the yield of the 2-(N-phenylamino)benzoic acid is reduced. These findings are summarized in Table 6.

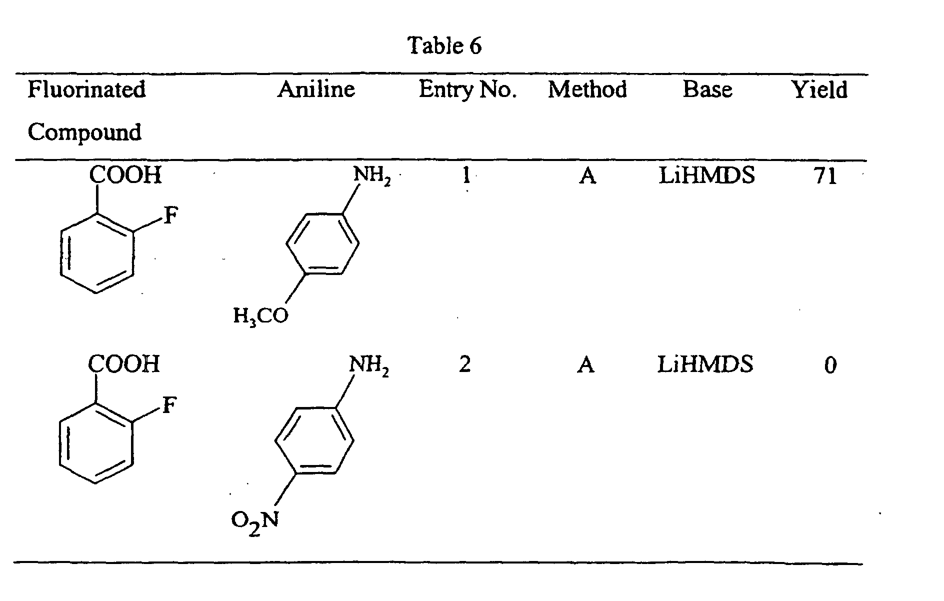

In addition, the presence of electron withdrawing groups on the benzoic acid can enhance the reactivity of the benzoic acid with the aniline, and therefore, increase the yield of the resulting 2-(N-phenylamino) benzoic acid.

The reaction is typically run in a solvent. The most preferred solvents are polar, aprotic solvents such as tetrahydrofuran and diethyl ether. The temperature of the reaction is selected to provide for the greatest yield. A suitable temperature can be easily selected by one skilled in the art. A preferred temperature range is -78°C to 25°C.

The examples presented herein are intended to be illustrative of the invention.

## Claims

1. A method for making 2-(N-phenylannino)benzoic acids, the method comprising the step of reacting a benzoic acid having the Formula I 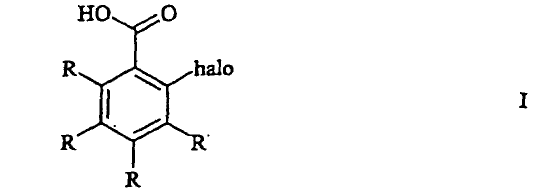 and an aniline having the Formula II 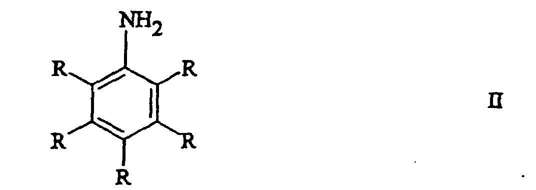 with an alkaline metal hexamethyldisilazide to form a 2-(N-phenylamino)benzoic acid having the Formula III 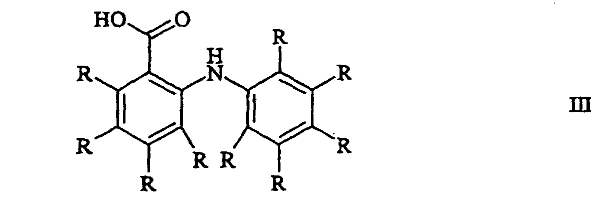 wherein
each R is independently hydrogen, halogen, C₁-C₆alkyl, -OC₁-C₆ alkyl, CN, or NO₂ with the proviso that R on the aniline is not NO₂.

2. The method of Claim 1 wherein the alkaline metal hexamethyldisilazide is lithium hexamethyldisilazide.

3. The method of Claim 1 wherein the alkaline metal hexamethyldisilazide is 3 equivalents or more with respect to the benzoic acid.

4. The method of Claim 1 wherein the halo substituent in the 2 position of the benzoic acid is fluorine.

5. The method of Claim 1 wherein the reaction is carried out at about -78°C to 25°C in a polar aprotic solvent.

6. The method of Claim 5 wherein the polar aprotic solvent is tetrahydrofuran.

7. The method of Claim 1 wherein the benzoic acid is 2,3,4,-trifluorobenzoic acid and the aniline is 2-chloro-4-iodoaniline.

8. The method of Claim 1 wherein the benzoic acid and the aniline are resent in a 1:1 molar ratio.

9. The method of Claim 1 wherein one or more of the substituents, R, on the aniline is an electron donating group.

10. The method of Claim 9 wherein the electron donating group is -OCH₃.

## Patentansprüche

1. Verfahren zur Herstellung von 2-(N-Phenylamino)-benzoesäuren, umfassend den Schritt der Umsetzung einer Benzoesäure der Formel I 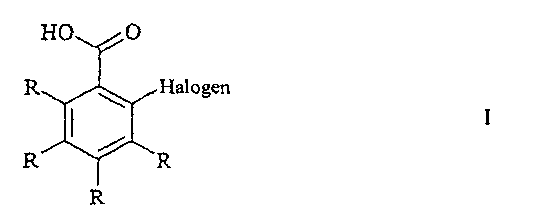 und eines Anilins der Formel II 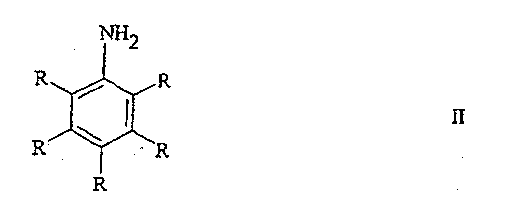 mit einem Alkalimetallhexamethyldisilazid zu einer 2-(N-Phenylamino)benzoesäure der Formel III 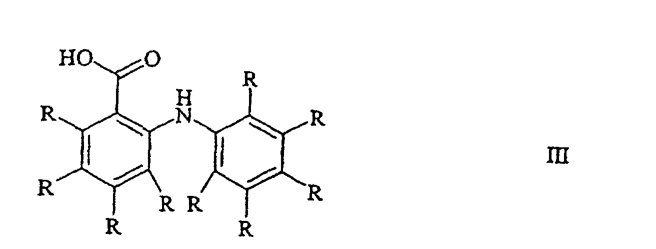 umsetzt, wobei jedes R jeweils unabhängig für Wasserstoff, Halogen, C₁-C₆-Alkyl, -O-C₁-C₆-Alkyl, CN oder NO₂ steht, mit der Maßgabe, daß R an dem Anilin nicht für NO₂ steht.

2. Verfahren nach Anspruch 1, bei dem es sich bei dem Alkalimetallhexamethyldisilazid um Lithiumhexamethyldisilazid handelt.

3. Verfahren nach Anspruch 1, bei dem das Alkalimetallhexamethyldisilazid in einer Menge von 3 Äquivalenten oder mehr, bezogen auf die Benzoesäure, verwendet wird.

4. Verfahren nach Anspruch 1, bei dem es sich bei dem Halogensubstituenten in der 2-Stellung der Benzoesäure um Fluor handelt.

5. Verfahren nach Anspruch 1, bei dem die Umsetzung bei etwa -78°C bis 25°C in einem polaren aprotischen Lösungsmittel durchführt wird.

6. Verfahren nach Anspruch 5, bei dem es sich bei dem polaren aprotischen Lösungsmittel um Tetrahydrofuran handelt.

7. Verfahren nach Anspruch 1, bei dem es sich bei der Benzoesäure um 2,3,4-Trifluorbenzoesäure und bei dem Anilin um 2-Chlor-4-iodanilin handelt.

8. Verfahren nach Anspruch 1, bei dem die Benzoesäure und das Anilin in einem Molverhältnis von 1:1 vorliegen.

9. Verfahren nach Anspruch 1, bei dem es sich bei einem oder mehreren der Substituenten R an dem Anilin um eine elektronenliefernde Gruppe handelt.

10. Verfahren nach Anspruch 9, bei dem es sich bei der elektronenliefernden Gruppe um -OCH₃ handelt.

## Revendications

1. Procédé de préparation d'acides 2-(N-phénylamino)benzoïques, le procédé comprenant l'étape de réaction d'un acide benzoïque présentant la formule I 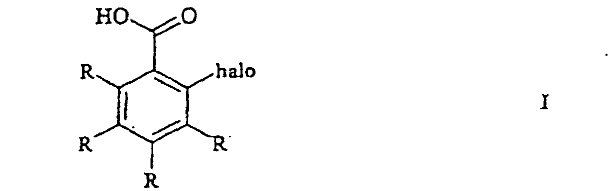 et d'une aniline présentant la formule II 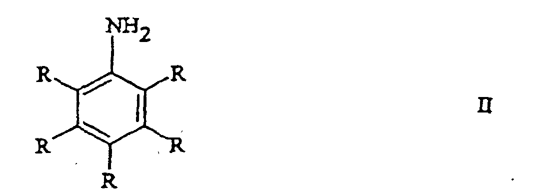 avec un hexaméthyldisilazane de métal alcalin de manière à former un acide 2-(N-phénylamino)benzoïque présentant la formule III 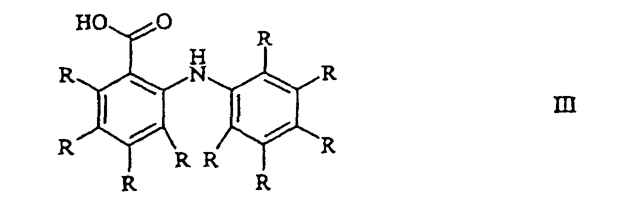 dans laquelle
chaque R représente, indépendamment, hydrogène, halogène, alkyle en C₁ à C₆, -O-alkyle en C₁ à C₆, CN ou NO₂ à condition que R sur l'aniline ne représente pas NO₂.

2. Procédé selon la revendication 1, dans lequel l'hexaméthyldisilazane de métal alcalin est l'hexaméthyldisilazane de lithium.

3. Procédé selon la revendication 1, dans lequel l'hexaméthyldisilazane de métal alcalin est présent à raison de 3 équivalents ou plus par rapport à l'acide benzoïque.

4. Procédé selon la revendication 1, dans lequel le substituant halogène en 2ème position de l'acide benzoïque est le fluor.

5. Procédé selon la revendication 1, dans lequel la réaction est effectuée à environ -78°C jusqu'à 25°C dans un solvant polaire aprotique.

6. Procédé selon la revendication 5, dans lequel le solvant polaire aprotique est le tétrahydrofuranne.

7. Procédé selon la revendication 1, dans lequel l'acide benzoïque est l'acide 2,3,4-trifluorobenzoïque et l'aniline est la 2-chloro-4-iodoaniline.

8. Procédé selon la revendication 1, dans lequel l'acide benzoïque et l'aniline sont présents dans un rapport molaire 1:1.

9. Procédé selon la revendication 1, dans lequel un ou plusieurs des substituants R sur l'aniline sont un groupe donneur d'électrons.

10. Procédé selon la revendication 9, dans lequel le groupe donneur d'électrons est -OCH₃.
